# EUROPÄISCHE PATENTSCHRIFT

(11) **EP 0 958 796 B1**
(45) Veröffentlichungstag und Bekanntmachung des Hinweises auf die Patenterteilung: **15.10.2003**
(21) Anmeldenummer: 99108461.7
(22) Anmeldetag: 30.04.1999
(51) Int. Cl.: A61F 2/16

(54) **Intraokularlinse**
Intraocular lens
Lentille intraoculaire

(30) Priorität: 11.05.1998 DE 19821029; 09.11.1998 DE 19851478
(43) Veröffentlichungstag der Anmeldung: 24.11.1999
(73) Patentinhaber: Neuhann, Tobias Dr., 80331 München (DE)
(72) Erfinder: Neuhann, Tobias Dr., 80331 München (DE)
(74) Vertreter: Nöth, Heinz, Dipl.-Phys.

(56) Entgegenhaltungen:
- EP-A- 0 337 390
- EP-A- 0 507 292
- EP-A- 0 599 457
- EP-A- 0 916 320
- FR-A- 2 661 816
- US-A- 5 098 443
- US-A- 5 171 320

## Beschreibung

Die Erfindung betrifft eine Intraokularlinse nach dem Oberbegriff des Patentanspruches 1.

Aus EP-A-0 337 390 ist eine derartige Intraokularlinse bekannt, welche als Hinterkammerlinse mit ihrem Linsenkörper in einen nach einer extrakapsulären Operation im Auge verbliebenen Kapsel implantiert werden kann. Bei der bekannten Intraokularlinse sind wenigstens zwei Linsenkörper für die Akkommodation des Auges auf unterschiedliche Entfernungen beweglich zueinander angeordnet. Hierfür ist am äquatorrealen Rand des Linsenkörpers ein nach außen offenes umlaufendes Profil vorgesehen, welches elastisch verformbar ist und von zwei abgerundeten Profilrändern begrenzt wird.

Bei einer extrakapsulären Kataraktoperation wird ein rundes Stück der vorderen Linsenkapsel entfernt und der Kern exprimiert. Die zurückgebliebene Linsenrinde wird durch ein Saug-Spül-System von der intakt gebliebenen Hinterkapsel abgesaugt. Anschließend wird eine Hinterkammerlinse als Intraokularlinse an die Stelle der entfernten natürlichen Augenlinse implantiert. Mit Hilfe bekannter Haptikmittel, beispielsweise elastischen Schlingen oder dergl., stützt sich die implantierte Linse im Kapselsack, insbesondere im Äquatorbereich des Kapselsackes, ab.

Häufig ergeben sich später entstehende Trübungen durch Nachstarbildung bzw. Fibrose. Das Fibrose-Material entsteht aus Epithelzellen, welche im Äquatorbereich des Kapselsackes verblieben sind. Diese Zellen haben das Bestreben, sich über die Fläche der Hinterkapsel auszubreiten, wodurch die Eintrübungen an der Hinterkapsel entstehen.

Aufgabe der Erfindung ist es, eine Intraokularlinse der eingangs genannten Art zu schaffen, durch welche eine Nachstarbildung bzw. Fibrose an der Hinterkapsel vermieden wird.

Diese Aufgabe wird erfindungsgemäß durch die kennzeichnenden Merkmale des Patentanspruches 1 gelöst.

Die spitz zulaufenden Profilränder sind in der Weise ausgestaltet, dass beide oder wenigstens der hintere Profilrand im implantierten Zustand der Intraokularlinse an den dem Kapselsackäquator benachbarten Kapselsackbereichen anliegen bzw. anliegt.

Dadurch, dass die Profilränder, nämlich ein vorderer und/oder hinterer am Äquator des Linsenkörpers umlaufender Profilrand zu beiden Seiten des Kapselsackäquators am Kapselsackmaterial anliegen, bilden diese Ränder bzw. bildet dieser Rand eine Barriere gegen das Ausbreiten der im Kapselsackäquator verbliebenen Epithelzellen, insbesondere auf die Fläche der Hinterkapsel. Nachwachsende Epithelzellen im Äquator des Kapselsackes werden von der konkaven Randgestaltung des Linsenkörpers eingefangen und an einer Ausbreitung gehindert.

Hierdurch ergibt sich der zusätzliche Vorteil einer Verankerung der Linse im Randbereich an der Innenseite des Kapselsackes.

In vorteilhafter Weise kann das umlaufende konkave Randprofil des Linsenkörpers eine Halteeinrichtung für Additive, welche insbesondere eine Wucherung der verbliebenen restlichen Epithelzellen bewirken, bilden.

Anhand der Figuren wird an Ausführungsbeispielen die Erfindung näher erläutert.
Es zeigt:
- Fig. 1:: ein erstes Ausführungsbeispiel;
- Fig. 2:: ein zweites Ausführungsbeispiel; und
- Fig. 3:: ein drittes Ausführungsbeispiel.

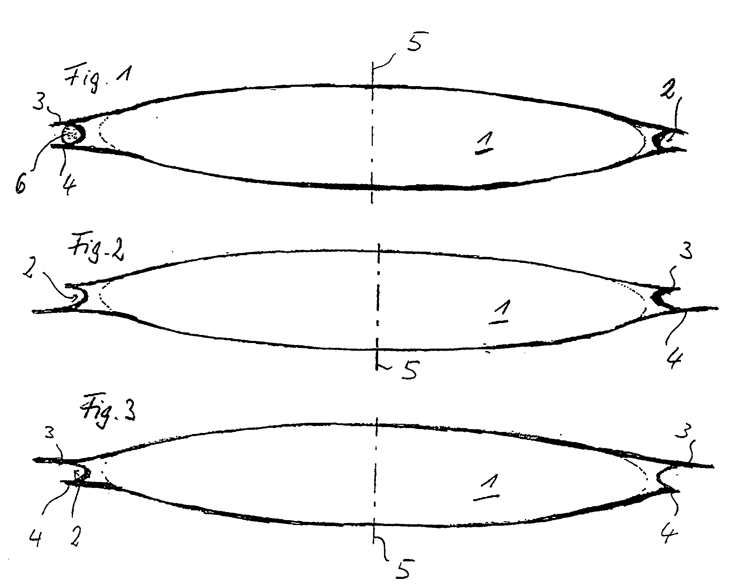

Die Darstellungen in den Figuren 1 und 3 zeigen Meridianschnitte durch die Linsenkörper der Ausführungsbeispiele.

Bei den Ausführungsbeispielen sind die äquatorialen Ränder eines jeweiligen Linsenkörpers 1 zu einem nach außen offenen umlaufenden konkaven Profil 2 geformt. Dieses konkave Randprofil 2 wird von zwei Profilrändern 3 und 4 begrenzt. Beim Ausführungsbeispiel der Fig. 1 besitzen die Profilränder 3 und 4 einen gleichen radialen Abstand von einer durch die Mitte des Linsenkörpers 1 verlaufende optische Linsenachse 5. Bei den Ausführungsbeispielen der Figuren 2 und 3 besitzen die Profilränder 3 und 4 unterschiedliche Abstände von der optischen Linsenachse 5. Beim Ausführungsbeispiel der Fig. 2 besitzt der vordere Profilrand 3 zur optischen Linsenachse 5 einen kürzeren Abstand als der hintere Profilrand 4. Beim Ausführungsbeispiel der Fig. 3 besitzt der vordere Profilrand 3 einen größeren Abstand zur optischen Linsenachse 5 als der hintere Profilrand 4. Der vordere Profilrand 3 liegt im implantierten Zustand näher zur Augenvorderfläche (Hornhautoberfläche) als der hintere Profilrand 4.

Das Profil 2 kann eine Halteeinrichtung bilden für Additive, die beispielsweise ein Zellwachstum (Epithelzellwachstum) behindern. Die Additive können in einem porösen ringförmigen Körper 6 vorgesehen sein, der in den konkaven Raum des Profils 2 eingelegt ist und sich entlang dem gesamten äquatorialen Rand des Linsenkörpers 1 erstreckt.

Die Profilränder 3 und 4, welche ausgehend vom Linsenkörper 1 spitz zulaufen und das konkave Profil 2 begrenzen, liegen im implantierten Zustand des Linsenkörpers 1 an Kapselsackbereichen an, welche dem Kapselsackäquator benachbart sind und zu beiden Seiten des Kapselsackäquators liegen. Hierdurch bilden die Profilränder 3 und 4 und insbesondere der hintere Profilrand 4 eine mechanische Barriere gegen eine sich insbesondere an der Fläche der Hinterkapsel ausbreitenden Wucherung von Epithelzellen, welche im Bereich des Äquators des Kapselsackes bei der extrakapsulären Kataraktoperation zurückgeblieben sind. Kapselfibrose an der Hinterkapsel und sich daraus ergebende Trübungen der Hinterkapsel, insbesondere im optisch wirksamen Bereich des Linsenkörpers 1 hinter der Pupille des Auges, werden hierdurch verhindert. Falls eine Wucherung von Restepithelzellen im Äquatorbereich des Kapselsackes sich ergibt, wird diese auf den umlaufenden konkaven Raum des Randprofils 2 am Äquator des Linsenkörpers 1 begrenzt.

Bei den in den Figuren dargestellten Ausführungsbeispielen besitzt das außen offene umlaufende konkave Profil, welches in den äquatorialen Rand des Linsenkörpers eingeformt ist, eine gewölbte Begrenzungsfläche. Natürlich können auch konkave Profile mit geradlinigen Begrenzungsflächen, die in einer oder mehreren Ecken zusammenlaufen, zum Einsatz kommen, um eine Ausbreitung der im Kapselsackäquator verbliebenen Epithelzellen zu verhindern.

## Patentansprüche

1. Intraokularlinse, welche als Hinterkammerlinse mit ihrem Linsenkörper in einen nach einer extrakapsulären Operation im Auge verbliebenen Kapselsack implantierbar ist, wobei der äquatoriale Rand des Linsenkörpers (1) ein nach außen offenes umlaufendes konkaves Profil (2) aufweist, das von zwei Profilrändern (3, 4) begrenzt ist, **dadurch gekennzeichnet, dass** die beiden Profilränder (3, 4) ausgehend vom Linsenkörper (1) spitz zulaufen und so ausgebildet sind, dass von den beiden Profilrändern (3, 4) zumindest der hintere Profilrand (4) im implantierten Zustand an dem dem Kapselsackäquator benachbarten Kapselsackbereich auf der Hinterkapsel anlegbar ist.

2. Intraokularlinse nach Anspruch 1,
**dadurch gekennzeichnet, dass** die beiden Profilränder (3, 4) den gleichen radialen Abstand von der optischen Linsenachse (5) haben.

3. Intraokularlinse nach Anspruch 1,
**dadurch gekennzeichnet, dass** die beiden Profilränder (3, 4) unterschiedliche radiale Abstände von der optischen Linsenachse (5) aufweisen.

4. Intraokularlinse nach einem der Ansprüche 1 bis 3,
**dadurch gekennzeichnet, dass** das umlaufende Profil (2) eine Halteeinrichtung für Additive bildet.

## Claims

1. An intraocular lens which is implantable as a posterior chamber lens with its lens body in a capsule sac remaining in the eye after an extracapsular operation, wherein the equatorial edge of the lens body (1) has an outwardly open peripheral concave profile (2) which is delimited by two profile edges (3, 4), **characterised in that** the two profile edges (3, 4) converge towards a point from the lens body (1) and are of such a configuration that of the two profile edges (3, 4) at least the rear profile edge (4) in the implanted condition can be caused to bear against the capsule sac region adjacent to the capsule sac equator on the posterior capsule.

2. An intraocular lens according to claim 1 **characterised in that** the two profile edges (3, 4) are at the same radial spacing from the optical axis (5) of the lens.

3. An intraocular lens according to claim 1 **characterised in that** the two profile edges (3, 4) are at different radial spacings from the optical axis (5) of the lens.

4. An intraocular lens according to one of claims 1 to 3 **characterised in that** the peripherally extending profile (2) forms a holding device for additives.

## Revendications

1. Lentille intraoculaire, qui peut être implantée par son corps de lentille, en tant que lentille de chambre postérieure, dans un sac capsulaire resté dans l'oeil après une opération extra-capsulaire, le bord équatorial du corps de lentille (1) présentant un profil concave périphérique (2) ouvert vers l'extérieur, délimité par deux bords profilés (3, 4), **caractérisée en ce que** les deux bords profilés (3, 4) se terminent en pointe à partir du corps de lentille (1) et sont configurés de telle sorte que, sur les deux bords profilés (3, 4), au moins le bord profilé arrière (4) peut s'appliquer sur la capsule postérieure à l'état implanté sur la région du sac capsulaire adjacente à l'équateur du sac capsulaire.

2. Lentille intraoculaire suivant la revendication 1, **caractérisée en ce que** les deux bords profilés (3, 4) présentent la même distance radiale par rapport à l'axe lenticulaire optique (5).

3. Lentille intraoculaire suivant la revendication 1, **caractérisée en ce que** les deux bords profilés (3, 4) présentent des distances radiales différentes par rapport à l'axe lenticulaire optique (5).

4. Lentille intraoculaire suivant l'une des revendications 1 à 3, **caractérisée en ce que** le profil périphérique (2) forme un dispositif de retenue pour des additifs.
